# EUROPÄISCHE PATENTSCHRIFT

(11) **EP 1 379 854 B1**
(45) Veröffentlichungstag und Bekanntmachung des Hinweises auf die Patenterteilung: **19.07.2006**
(21) Anmeldenummer: 02737964.3
(22) Anmeldetag: 16.04.2002
(51) Int. Cl.: G01N 3/20, G01N 33/34

(54) **MESSEN DES FALTVERHALTENS ODER FALTWIDERSTANDES VON ZUSCHNITTEN**
METHOD AND DEVICE FOR MEASURING BLANKS
PROCEDE ET DISPOSITIF POUR MESURER DES PIECES DECOUPEES

(30) Priorität: 20.04.2001 DE 10119634
(43) Veröffentlichungstag der Anmeldung: 14.01.2004
(73) Patentinhaber: Focke & Co. (GmbH & Co. KG), 27283 Verden (DE)
(72) Erfinder: FOCKE, Heinz, 27283 Verden (DE); STILLER, Martin, 27283 Verden (DE); SCHMIDT, Jens, 28879 Grasberg (DE); KOLLER, Lars, 30459 Hannover (DE)
(74) Vertreter: Bolte, Erich
(86) Internationale Anmeldenummer: PCT/EP2002/004194
(87) Internationale Veröffentlichungsnummer: WO 2002/086458

(56) Entgegenhaltungen:
- WO-A-02/066957
- DE-A- 2 745 182
- US-A- 4 358 962
- US-A- 4 753 113
- US-A- 5 574 227
- US-A- 5 955 680

## Beschreibung

Die Erfindung betrifft eine Vorrichtung zum Messen des Faltverhaltens bzw. des Faltwiderstands von Zuschnitten und deren durch geprägte Längs- oder Querfaltlinien abgegrenzte Faltlappen, insbesondere von Zuschnitten aus (dünnem) Karton für die Herstellung von Zigarettenpackungen des Typs Klappschachtel, in einer Prüfeinheit mit einer Halterung zur Aufnahme des Zuschnitts zwischen Halteorganen, wobei ein freier, nicht in der Halterung fixierter Teil des Zuschnitts relativ zu dem übrigen Teil verschwenkbar und die in einem oder mehreren Faltlappen erzeugte Rückstellkraft mittels Messtaster messbar ist.

Eine bekannte Vorrichtung zum Prüfen von Packmaterial (US 4 358 962) ist mit einer Halterung für den zu prüfenden Zuschnitt versehen, die aus einer unteren Tragplatte und einer Blattfeder zum Fixieren des Zuschnitts besteht. Ein zu prüfender Lappen ragt über die untere Tragplatte hinaus, derart, dass eine durch Prägen gebildete Faltlinie frei liegt. Der zu prüfende Lappen ist durch einen manuell zu betätigenden Arm schwenkbar, wobei der Arm mittels Sauger an einer nach oben gerichteten Seite des Lappens fixiert ist. Eine so ausgebildete Prüfvorrichtung ermöglicht keine exakte Prüfung des Faltverhaltens des Zuschnitts.

Der Erfindung liegt die Aufgabe zugrunde, eine Prüfvorrichtung für Verpackungsmaterial, insbesondere aus dünnem Karton, vorzuschlagen, mit der eine sensible Prüfung des Faltverhaltens von Zuschnitten mit durch geprägte Faltlinien definierten Faltlappen möglich ist.

Zur Lösung dieser Aufgabe ist die erfindungsgemäße Vorrichtung durch folgende Merkmale gekennzeichnet:
a) die Halterung besteht aus zwei Haltebacken zwischen denen ein Teilbereich des Zuschnitts fixiert ist,
b) die Halterung, nämlich deren Haltebacke, weist eine Ausrichtkante auf, an der die Faltlinie während des Prüfvorgangs anliegt,
c) die Ausrichtkante ist an die Form der zu prüfenden Faltlinie bzw. der Packungskante angepasst, nämlich durch einen Rücksprung für eine geprägte Faltlinie oder durch eine Rundung für die Messung von Zuschnitten mit runden Packungskanten oder durch eine Abschrägung für die Messung von Zuschnitten mit abgeschrägten Packungskanten ,
d) die Halterung ist zur Durchführung der Prüfung drehbar, wobei ein sich außerhalb des Bereichs der Haltebacken befindlicher Teil des Zuschnitts an dem Messtaster anliegt,
e) eine Drehachse der Halterung bzw. der Haltbacken erstreckt sich entlang der Ausrichtkante.

Die Prüfvorrichtung bzw. deren Klemmorgane zum Erfassen des Zuschnitts weist bzw. weisen eine besonders gestaltete Ausrichtkante auf, die der zu prüfenden Faltkante zugeordnet und auf diese abgestimmt ist. Die Prüfung wird unter Berücksichtigung der Struktur der Falt- bzw. Packungskante durchgeführt. Bei der Prüfung von Zuschnitten für Klappschachteln mit Rundkanten oder Schrägkanten befindet sich erfindungsgemäß eine Drehachse der Klemmbacken in einem gedachten Mittelpunkt der Rundkante bzw. Schrägkante.

Eine weitere Besonderheit besteht darin, dass die Prüfvorrichtung hinsichtlich der Zuführung und des Abtransports der zu prüfenden Zuschnitte automatisiert ist.

Weitere Einzelheiten und Merkmale der Erfindung werden nachfolgend anhand der Zeichnungen näher erläutert. Es zeigt:

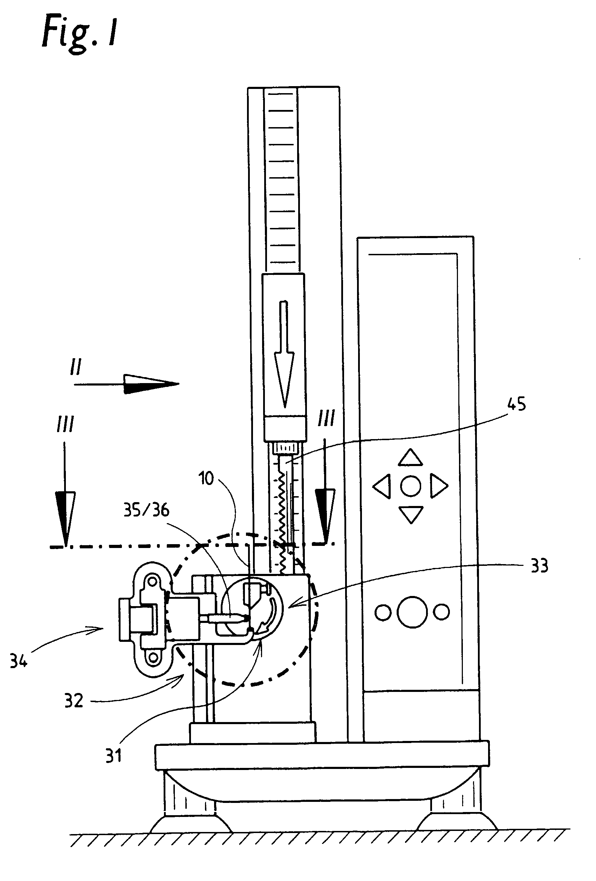

- Fig. 1: eine Vorrichtung zum Prüfen von Zuschnitten in Seitenansicht,

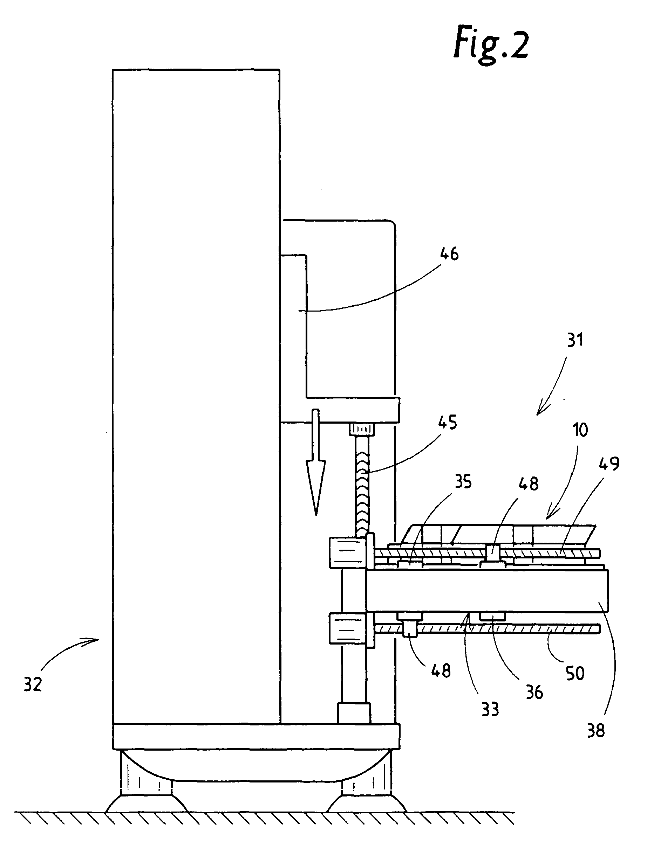

- Fig. 2: die Vorrichtung gemäß Fig. 1 in Seitenansicht entsprechend Pfeil II in Fig. 1,

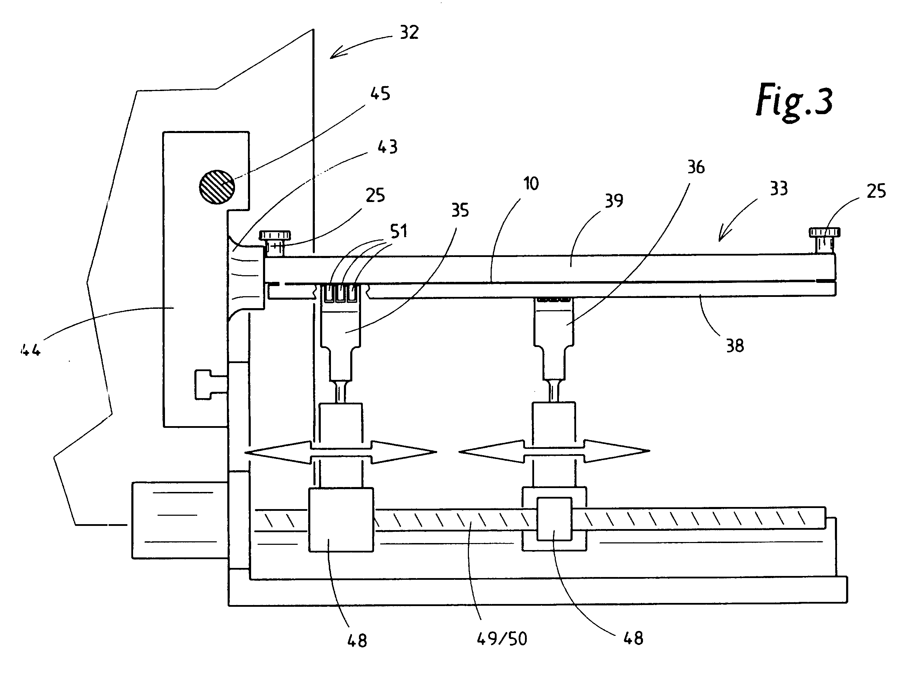

- Fig. 3: die Vorrichtung im Horizontalschnitt bei vergrößertem Maßstab in einer Schnittebene III-III der Fig. 1,
- Fig. 4: die Einzelheit der Prüfvorrichtung mit einem Zuschnitt für Klappschachteln in Seitenansicht,
- Fig. 5: die Prüfvorrichtung in Frontansicht mit einem Zuschnitt in Ausgangsstellung des Prüfverfahrens,
- Fig. 6: eine Darstellung entsprechend Fig. 5 in einer Phase des Prüfverfahrens,
- Fig. 7: eine Einzelheit aus Fig. 6 bei vergrößertem Maßstab,
- Fig. 8 bis Fig. 10: die Prüfung eines Zuschnitts für die Herstellung von Rundkanten-Packungen in aufeinanderfolgenden Prüfphasen,
- Fig. 11: eine Darstellung analog Fig. 9 bzw. Fig. 10 für die Prüfung eines Zuschnitts zum Herstellen von Packungen mit abgeschrägten Kanten,
- Fig. 12: eine Vorrichtung in Draufsicht zur automatischen Handhabung von Zuschnitten bei der Prüfung derselben,
- Fig. 13: die Vorrichtung gemäß Fig. 12 in Seitenansicht,
- Fig. 14: eine Queransicht der Vorrichtung gemäß Fig. 13 in der Ebene XIV - XIV der Fig. 13,
- Fig. 15: die Ansicht gemäß Fig. 14 bei veränderter Stellung von Organen.

In den Zeichnungen ist das bevorzugte Anwendungsgebiet einer vorliegenden Prüfvorrichtung gezeigt, nämlich die Überprüfung von Zuschnitten 10 für Packungen des Typs Klappschachtel bzw. Hinge-Lid. Der Zuschnitt 10 besteht aus Bereichen zur Bildung eines (unteren) Schachtelteils 11 und eines Deckels 12. Packungswände sind durch Längsfaltlinien und Querfaltlinien voneinander abgegrenzt, und zwar für eine Schachtel-Vorderwand 13, einen Boden 14, eine Schachtel-Rückwand 15, eine Deckel-Rückwand 16, eine Stirnwand 17 und eine Deckel-Vorderwand 18. Diese Bereiche des Zuschnitts 10 sind durch (parallele) Querfaltlinien 19 voneinander abgegrenzt. Zu beiden Seiten der vorgenannten Zuschnittbereiche sind Faltlappen gebildet, und zwar äußere Seitenlappen 20 sowie mit diesen korrespondierende innere Seitenlappen 21. Diese liegen bei der fertigen Packung aneinander und bilden bei teilweiser oder vollständiger Überdeckung Packungs-Seitenwände. Entsprechende Faltlappen sind im Bereich des Deckels 12 vorgesehen, nämlich äußere Deckel-Seitenlappen 22 und innere Deckel-Seitenlappen 23, jeweils für Deckel-Seitenwände. Die vorgenannten Seitenlappen 20..23 sind von den angrenzenden Bereichen des Zuschnitts 10 durch Längsfaltlinien 24 abgegrenzt.

Als seitliche Faltlappen sind weiterhin Boden-Ecklappen 28 und Deckel-Ecklappen 29 vorgesehen. Diese sind jeweils mit dem inneren Seitenlappen 21 bzw. dem inneren Deckel-Seitenlappen 23 verbunden und über die entsprechend fortgesetzten Querfaltlinien 19 begrenzt. Die Ecklappen 28, 29 sind durch einen L-förmigen Stanzschnitt 30 von anderen benachbarten Bereichen des Zuschnitts 10 abgetrennt.

Die Prüfvorrichtung für Zuschnitte 10 weist eine Prüfeinheit 31 auf, die an einem Traggestell 32 angebracht ist. Die Prüfeinheit 31 besteht aus einer Halterung 33 für den zu prüfenden Zuschnitt 10 und einer Messeinrichtung 34 mit mehreren, zum Beispiel zwei Messtastem 35, 36.

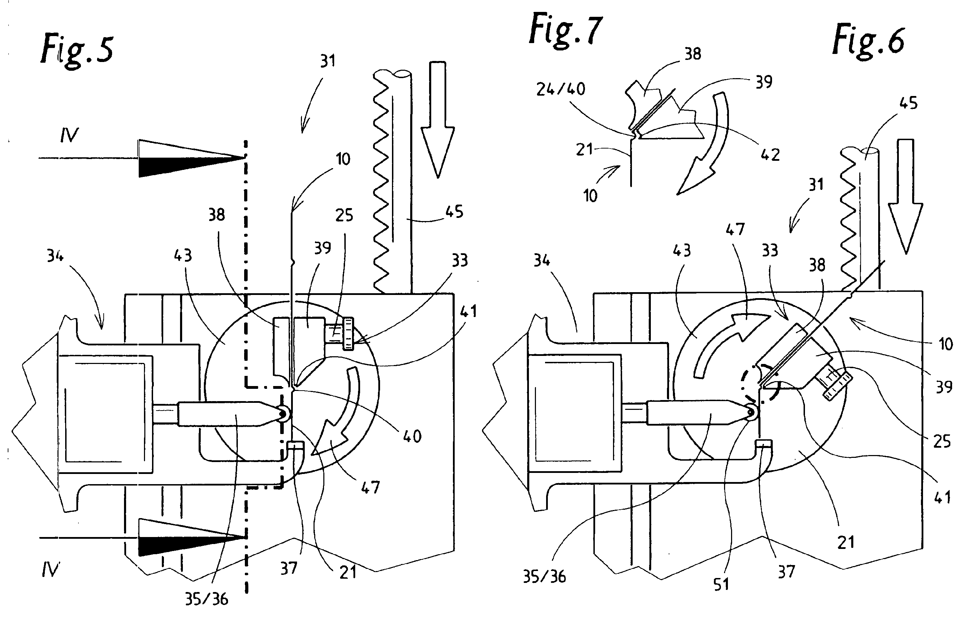

Der Zuschnitt 10 wird in der Prüfeinheit 31 in einer aufrechten Ebene positioniert (z.B. Fig. 5). Die Längserstreckung des Zuschnitts 10 ist dabei horizontal gerichtet, die vorrangig zu prüfenden Faltlappen 20, 21.. sind nach oben oder unten gerichtet. Eine freie Randkante des Zuschnitts 10 ist auf einem exakten Justierorgan, nämlich auf einem langgestreckten Stützanschlag 37, abgestützt und so ausgerichtet. Die Halterung 33 weist Halteorgane auf, nämlich zwei langgestreckte, stegartige Haltebacken 38, 39, zwischen denen der Zuschnitt 10 Aufnahme findet. Die Haltebacken 38, 39 sind in einem mittleren Bereich des Zuschnitts 10 wirksam, der durch Schachtel-Vorderwand 13, Boden 14, Schachtel-Rückwand 15 etc. definiert ist. Bei dem gezeigten Ausführungsbeispiel soll eine (untere) Längsfaltlinie 24, die zugleich Packungskante ist, hinsichtlich des Faltverhaltens überprüft werden. Untere Ränder der Haltebacken 38, 39 sind auf diese Faltlinie 24 ausgerichtet, insbesondere eine Ausrichtkante 41 des Haltebacken 39, um die der Zuschnitt 10 gefaltet wird. Die Ausrichtkante 41 ist entsprechend der Form der zu prüfenden Faltlinie bzw. Packungskante in besonderer Weise konturiert, nämlich mit einem Rücksprung 42 versehen (Fig. 7), der der Form der durch Prägen des Materials gebildeten Faltlinie 24 entspricht. Zur Prüfung dieser Faltlinie 24 wird ein Teil des Zuschnitts 10 relativ zu einem anderen Teil verschwenkt.

Die Halterung 33 ist so ausgebildet, dass zwischen den Halteorganen, nämlich Haltebacken 38, 39, ein Spalt gebildet ist, in den der Zuschnitt 10 zur Prüfung eingeführt und gehalten wird. Die korrekte Höhenstellung des Zuschnitts wird durch den Stützanschlag 37 gewährleistet, auf dem der Zuschnitt 10 mit einer freien, unteren Randkante aufliegt. Der Stützanschlag 37 ist in der Höhe verstellbar, um eine exakte Ausrichtung des Zuschnitts 10 bzw. der zu prüfenden Faltlinie auf die Ausrichtkante 41 zu ermöglichen.

Die Haltbacken 38, 39 sind relativ zueinander bewegbar, und zwar zur Fixierung des Zuschnitts 10 in der Prüfstellung. Bei dem gezeigten Ausführungsbeispiel ist eine Haltebacke 39 feststehend und die andere Haltebacke 38 quer bewegbar, bei dem gezeigten Ausführungsbeispiel durch Rändelschrauben 25. Der Zuschnitt 10 wird nach Einführung in die exakte Prüfstellung durch Betätigen der Rändelschrauben 25 zwischen den beiden Haltebakken 38, 39 fest eingespannt, so dass eine Relativbewegung zu den Halteorganen, insbesondere zu der Ausrichtkante 41 nicht möglich ist. Nun kann die Prüfung vollzogen werden. Die relativ zu einander bewegbaren Haltebacken sind durch quergerichtete Führungsstifte 26 geführt.

Die Halterung 33 ist zur Durchführung der Messung drehbar. Die beiden Haltebacken 38, 39 sind als Einheit drehbar, und zwar um die Ausrichtkante 41 als Drehpunkt bzw. Drehachse. Die Faltlinie 24 liegt demnach in der Drehachse. Zu diesem Zweck sind die Haltebacken 38, 39 als frei abstehende, auskragende Halteorgane an einem drehbaren Träger angebracht, nämlich an einer Tragscheibe 43. Diese ist in einem Gehäuse 44 gelagert. Die Drehbewegung wird durch einen gesonderten Antrieb bewirkt, und zwar durch eine auf- und abbewegbare Zahnstange 45, die mit einem Ritzel (nicht gezeigt) an der Tragscheibe 43 in Eingriff steht. Die Zahnstange 45 ist an einem auf- und abbewegbaren Schlitten 46 angebracht.

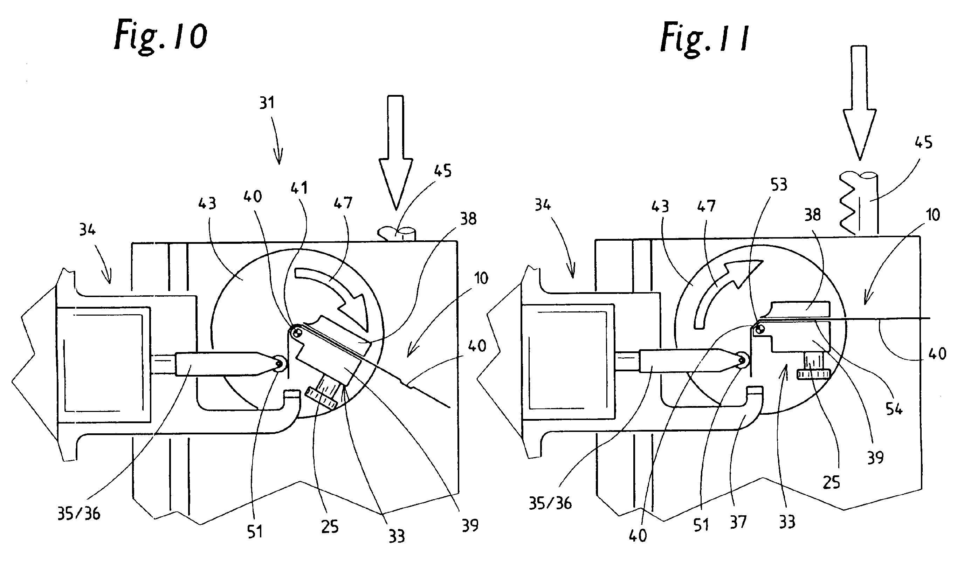

Zur Durchführung der Prüfung wird ein oberer Teil des Zuschnitts 10, nämlich oberhalb der Faltlinie 24, durch Drehen der Halterung 33 verschwenkt bis in eine Endstellung, die mindestens um 90° gegenüber der aufrechten Ausgangsstellung versetzt ist, vorzugsweise aber um einen Winkel von mehr als 90°, nämlich 120° (Fig. 10). Die Dreh- bzw. Schwenkbewegung erfolgt hier im Uhrzeigersinn gemäß Pfeil 47. Es wird dabei die Richtung einer Faltbewegung des betreffenden Teils des Zuschnitts berücksichtigt.

Zur Messung des Faltwiderstands ist auf der gegenüberliegenden, freien Seite des jeweils zu prüfenden Faltlappens, also unterhalb der Faltlinie 24, mindestens ein Messtaster 35, 36 angeordnet. Dieser erstreckt sich in horizontaler Ebene, wobei ein Tastende an dem Zuschnitt 10 bzw. am zu prüfenden Faltlappen anliegt.

Die Anordnung und Arbeitsweise der Messtaster 35, 36 ist eine Besonderheit. Es sind mehrere, im vorliegenden Falle zwei Messtaster 35, 36 vorgesehen, die in einer gemeinsamen, horizontalen Ebene nebeneinander angeordnet sind. Die Messtaster 35, 36 sind bewegbar, nämlich in einer horizontalen Richtung bzw. Ebene, um unterschiedliche Bereiche des Zuschnitts 10 messtechnisch zu erfassen. Für die Verstellbarkeit sind die Messtaster 35, 36 mit einer Spindelmutter 48 jeweils auf einer horizontal gerichteten, drehbaren Spindel 49, 50 gelagert. Durch Drehen der Spindel 49, 50 wird der jeweilige Messtaster 35, 36 in der einen oder anderen Richtung verstellt.

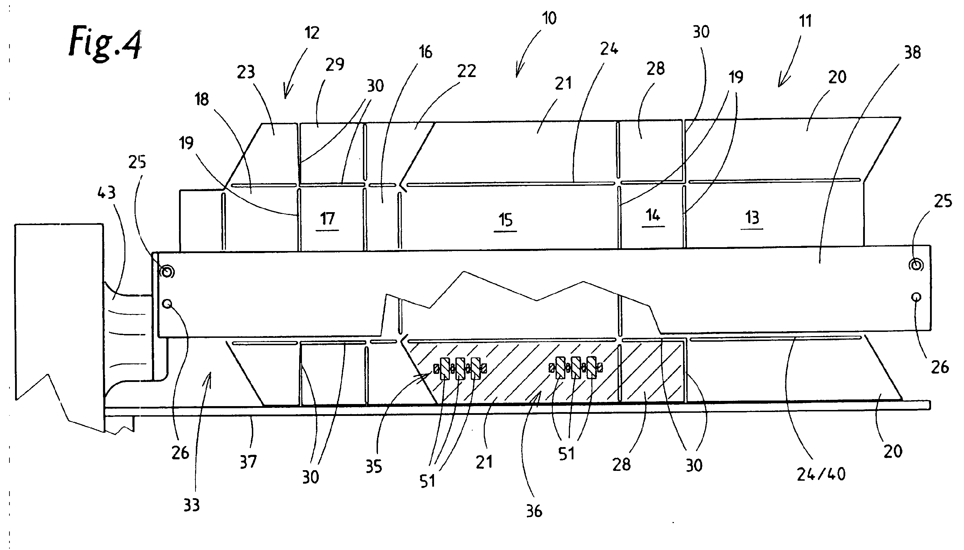

Bei der Position gemäß Fig. 4 wird eine falttechnische Einheit von Faltlappen überprüft, bestehend aus einem innenliegenden Seitenlappen 21 mit einem am Seitenlappen 21 angebrachten Boden-Ecklappen 28. Bei der Fertigung von Klappschachteln wird dieser in Fig. 4 schraffiert dargestellte Bereich als Einheit gefaltet. Unterbrechungen der Faltlinien, insbesondere der randseitigen Faltlinie 24, werden bei der Messung des Faltwiderstands berücksichtigt. Andere Mess- bzw. Falteinheiten sind die äußeren Seitenlappen 20, innerer Deckel-Seitenlappen 23 mit Deckel-Ecklappen 29 sowie der äußere Deckel-Seitenlappen 22.

Die beiden Messtaster 35, 36 sind in Bezug auf den Seitenlappen 21 so positioniert, dass eine gleichmäßige Kraftübertragung gegeben ist. Tastenden der Messtaster 35, 36 sind als Wälzkörper ausgebildet, bestehen nämlich aus (mehreren) Tastrollen 51. Im vorliegenden Falle sind drei Tastrollen 51 gleichachsig nebeneinander angeordnet. Diese Ausbildung des Tastendes ermöglicht eine reibungsfreie Übertragung der zu messenden Kräfte. Während der gesamten Schwenkbewegung des oberen Teils des Zuschnitts 10 aus einer Position gemäß Fig. 5 in eine Endstellung gemäß Fig. 10 werden die auftretenden Kräfte gemessen und über die Messeinrichtung einem Rechner zur Auswertung zugeführt.

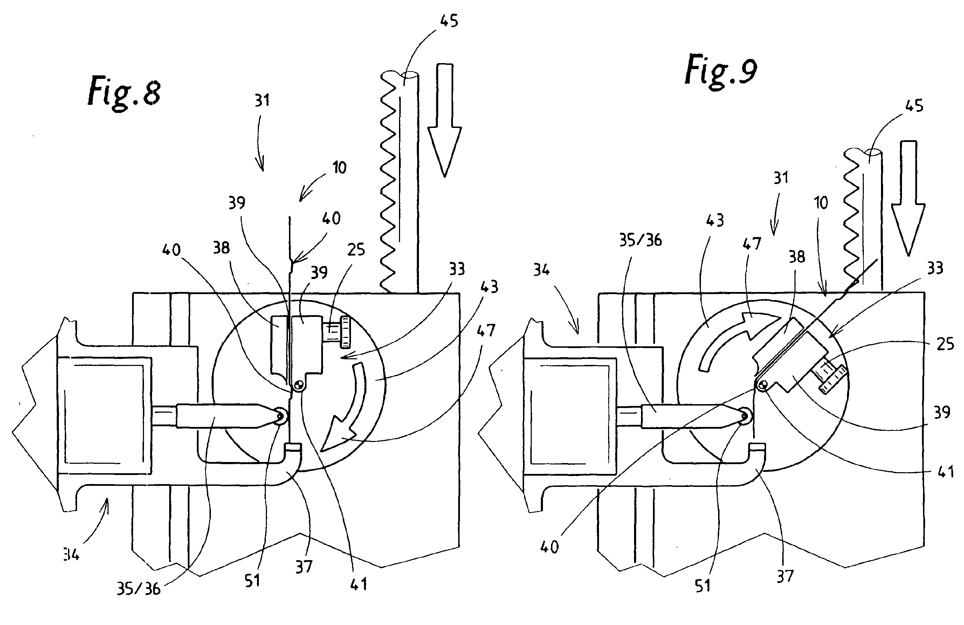

Eine Besonderheit ist die Messung von Zuschnitten 10 mit konturierten Packungskanten 40. In erster Linie handelt es sich um Klappschachteln des Typs Rundkanten (US 4 753 383) und des Typs Achteck (US 4 753 384). Fig 8 bis 10 zeigen Maßnahmen zur Messung des Faltverhaltens von Zuschnitten 10 für Rundkanten-Packungen. Der Zuschnitt 10 weist im Bereich der Packungskanten 40 eine Formgebung auf zur Bildung von Rundkanten. Insbesondere handelt es sich dabei um eine Gruppe von parallelen, dicht nebeneinanderliegenden Rillungen.

Die Halterung 33 ist auf die Ausbildung des Zuschnitts 10 mit Rundkanten ausgerichtet, und zwar im Bereich der Haltebacke 39. Deren Ausrichtkante 41 ist als Rundung 52 ausgebildet, und zwar als halbkreisförmige Rundung 52 mit einem Radius unter Anpassung an den Radius der zu schaffenden Rundkante (Fig. 10). Bei der Schwenkbewegung durch Drehen der Halterung 33 legt sich der Zuschnitt mit dem Bereich der Packungskante 40 um die gerundete Ausrichtkante 41, also an die Rundung 52 (Fig. 9, Fig. 10). Dabei ist von Bedeutung, dass die Drehachse der Halterung bzw. der Haltebacke 39 im Mittelpunkt der Rundkante bzw. der Rundung 52 der Ausrichtkante 41 liegt. Durch die Schwenkbewegung des bewegbaren Zuschnittteils wird der Zuschnitt 10 leicht angehoben, nämlich von dem Stützanschlag 37 abgehoben (Fig. 9, Fig. 10). Die Relativbewegung zum Messtaster 35, 36 vollzieht sich reibungsfrei wegen der Tastrollen 51.

Bei der Prüfung von Zuschnitten 10 für Packungen mit abgeschrägten Packungskanten 40 wird analog verfahren. Die Haltebacke 39 ist in diesem Falle im Bereich der Ausrichtkante 41 mit einer Abschrägung 53 versehen entsprechend einer Abschrägung bei Klappschachteln dieses Typs. Die Drehachse der Haltebacke 39 bzw. der Halterung 33 ist analog zu dem Ausführungsbeispiel gemäß Fig. 8 bis 10 positioniert, derart, dass eine gleichförmige Abwicklung des Zuschnitts 10 bzw. der Packungskante 40 um die Abschrägung 53 herum erfolgt. Die Drehachse ist in diesem Falle etwa 3 mm von Faltkanten der abgeschrägten Packungskante 40 entfernt.

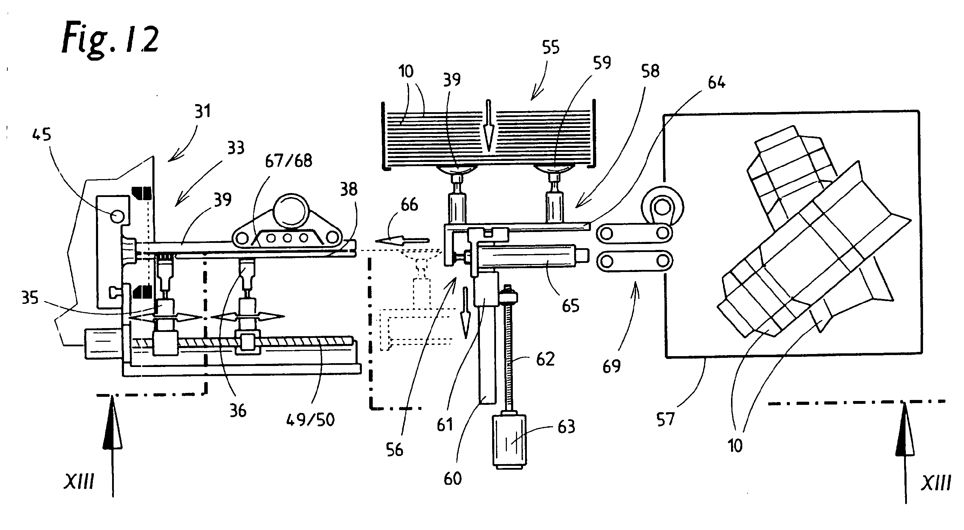

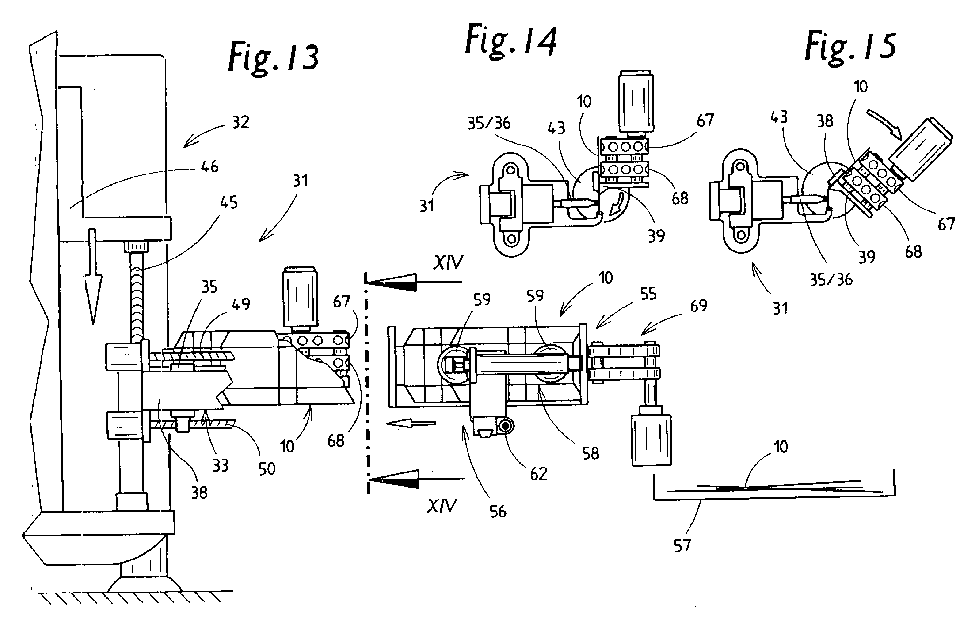

Die Prüfung der Zuschnitte 10 kann vollautomatisch durchgeführt werden, insbesondere mit in Fig. 12 und Fig. 13 gezeigten Maßnahmen. Die Zuschnitte werden dabei nacheinander einem Zuschnitt-Magazin 55 entnommen, und zwar durch eine (automatische) Handhabungseinheit 56. Von dieser werden die Zuschnitte 10 an eine Prüfeinheit 31 weitergegeben. Nach Durchführung der Prüfung werden die Zuschnitte 10 durch die Handhabungseinheit 56 einem Sammelbehälter 57 zugeführt.

Das Zuschnitt-Magazin 55 ist so angeordnet, dass die Zuschnitte 10 in aufrechter Ebene bereitliegen, mit der Längserstreckung in horizontaler Richtung. Der jeweils vordere Zuschnitt 10 wird durch einen Greifer entnommen, nämlich durch einen Saugkopf 58 mit zwei nebeneinander angeordneten Saughaltern 59.

Der Saugkopf 58 wird nach Erfassen eines Zuschnitts 10 vom Zuschnitt-Magazin 55 zurückbewegt. Zu diesem Zweck ist die Einheit mit dem Saugkopf 58 entlang einer horizontalen Führung 60 vom Zuschnitt-Magazin 55 wegbewegbar. Ein auf der Führung 60 bewegbarer Schlitten 61 ist durch ein Stellgetriebe verschiebbar, im vorliegenden Falle durch eine mit dem Schlitten 61 verbundene Spindel 62, die durch einen Servomotor 63 angetrieben wird.

Der Saugkopf 58 wird mit dem Zuschnitt 10 in eine Übergabeposition zur Prüfeinheit 31 bewegt (gestrichelt in Fig. 12). In dieser Stellung wird ein weiterer, quergerichteter Antrieb wirksam. Der Saugkopf 58 ist mit einer Schubstange 64 verbunden. Diese ist - mit dem Saugkopf 58 - quer verschiebbar, und zwar durch einen (pneumatischen) Betätigungszylinder 65. Dieser verschiebt den Saugkopf 58 - mit Zuschnitt 10 - in die Übergabeposition.

Zur Übergabe an die Prüfeinheit 31 werden die Zuschnitte 10 in der Prüfstellung in vertikaler Ebene bereitgehalten. Die Zuschnitte 10 werden dabei in Längsrichtung in den zwischen den beiden Halteorganen bzw. Haltebacken 38, 39 gebildeten Spalt 54 eingeführt, und zwar in horizontaler Förderrichtung entsprechend Richtungspfeil 66 in Fig. 12. Zur vollständigen und exakten Positionierung des Zuschnitts 10 im Bereich der Prüfeinheit 31 wird ein weiteres Förderorgan für den Zuschnitt 10 wirksam, nämlich ein aus zwei horizontalen Saugbändern 67, 68 bestehender Horizontalförderer. Die Saugbänder 67, 68 erfassen den Zuschnitt 10 in einem Bereich unmittelbar oberhalb der Halteorgane bzw. Haltebacken 38, 39 und bewegen den Zuschnitt 10 bis in die zur Prüfung erforderliche Endstellung unter Auflage auf dem Stützanschlag 37. Die Endstellung des Zuschnitts 10 in der Förderrichtung wird durch Sensoren überprüft, im vorliegenden Falle durch eine quergerichtete Lichtschranke 70, die bei Erreichen der Endstellung des Zuschnitts 10 den Transport beendet.

Die Halterung 33 für den Zuschnitt 10 kann bei diesem Ausführungsbeispiel für automatische Betriebsweise in der beschriebenen Form ausgebildet sein. Im vorliegenden Falle wird aber der Zuschnitt ohne Veränderung der Haltebacken 38, 39 zueinander durch den Horizontalförderer gehalten, also durch die beiden Saugbänder 67, 68. Die Haltebakken 38, 39 bleiben in einem Abstand von einander unter Bildung des (schmalen) Spalts 54. Bei der Prüfung des Zuschnitts wird diese gesonderte Halterung, nämlich der Horizontalförderer, mit den Haltebacken 38, 39 um die gemeinsame Drehachse gedreht. Der Förderer mit den Saugbändern 67, 68 ist zu diesem Zweck mit der Halterung 33 verbunden. Wie aus Fig. 14 und Fig. 15 ersichtlich, ist der Horizontalförderer an einer der Haltebacken angebracht, nämlich an der entsprechend vergrößerten bzw. verbreiterten Haltebacke 39, die die Ausrichtkante 41 aufweist.

Nach Prüfung des Zuschnitts 10 in der oben beschriebenen Weise wird dieser selbsttätig der Prüfeinheit 31 entnommen, und zwar durch Abfördern mit Hilfe der Saugbänder 67, 68. Diese übergeben den geprüften Zuschnitt an den Saugkopf 58, der bei gegenläufiger Förderbewegung des Betätigungszylinders 65 den Zuschnitt 10 bis in den Arbeitsbereich eines Abförderers 69 transportiert. Dieser besteht aus zwei Paaren von Fördergurten mit motorischem Antrieb. Der Abförderer 69 transportiert den geprüften Zuschnitt 10 in den Sammelbehälter 57.

### Bezugszeichenliste

- 10: Zuschnitt
- 11: Schachtelteil
- 12: Deckel
- 13: Schachtel-Vorderwand
- 14: Boden
- 15: Schachtel-Rückwand
- 16: Deckel-Rückwand
- 17: Stirnwand
- 18: Deckel-Vorderwand
- 19: Querfaltlinie
- 20: Seitenlappen (außen)
- 21: Seitenlappen (innen)
- 22: Deckel-Seitenlappen (außen)
- 23: Deckel-Seitenlappen (innen)
- 24: Längsfaltlinie
- 25: Rändelschraube
- 26: Führungsstift
- 28: Boden-Ecklappen
- 29: Deckel-Ecklappen
- 30: Stanzschnitt
- 31: Prüfeinheit
- 32: Traggestell
- 33: Halterung
- 34: Messeinrichtung
- 35: Messtaster
- 36: Messtaster
- 37: Stützanschlag
- 38: Haltebacke
- 39: Haltebacke
- 40: Packungskante
- 41: Ausrichtkante
- 42: Rücksprung
- 43: Tragscheibe
- 44: Gehäuse
- 45: Zahnstange
- 46: Schlitten
- 47: Pfeil
- 48: Spindelmutter
- 49: Spindel
- 50: Spindel
- 51: Tastrolle
- 52: Rundung
- 53: Abschrägung
- 54: Spalt
- 55: Zuschnitt-Magazin
- 56: Handhabungseinheit
- 57: Sammelbehälter
- 58: Saugkopf
- 59: Saughalter
- 60: Führung
- 61: Schlitten
- 62: Spindel
- 63: Servomotor
- 64: Schubstange
- 65: Betätigungszylinder
- 66: Richtungspfeil
- 67: Saugband
- 68: Saugband
- 69: Abförderer
- 70: Lichtschranke

## Patentansprüche

1. Vorrichtung zum Messen des Faltverhaltens bzw. des Faltwiderstands von Zuschnitten (10) und deren durch geprägte Längs- oder Querfaltlinien (24, 19) abgegrenzte Faltlappen, insbesondere von Zuschnitten (10) aus Karton für die Herstellung von Zigarettenpackungen des Typs Klappschachtel, in einer Prüfeinheit (31) mit einer Halterung (33) zur Aufnahme des Zuschnitts (10) zwischen Halteorganen, wobei ein freier, nicht in der Halterung (33) fixierter Teil des Zuschnitts relativ zu dem übrigen Teil verschwenkbar und die in einem oder mehreren Faltlappen erzeugte Rückstellkraft mittels Messtaster (35, 36) messbar ist, **gekennzeichnet durch** folgende Merkmale:
a) die Halterung (33) besteht aus zwei Haltebacken (38, 39) zwischen denen ein Teilbereich des Zuschnitts (10) fixiert ist,
b) die Halterung (33), nämlich deren Haltebacke (39), weist eine Ausrichtkante (41) auf, an der die Faltlinie (19, 24) während des Prüfvorgangs anliegt,
c) die Ausrichtkante (41) ist an die Form der zu prüfenden Faltlinie (24) bzw. der Packungskante (40) angepasst, nämlich **durch** einen Rücksprung (42) für eine geprägte Faltlinie (24) oder **durch** eine Rundung (52) für die Messung von Zuschnitten (10) mit runden Packungskanten (40) oder **durch** eine Abschrägung (53) für die Messung von Zuschnitten (10) mit abgeschrägten Packungskanten (40),
d) die Halterung (33) ist zur Durchführung der Prüfung drehbar, wobei ein sich außerhalb des Bereichs der Haltebacken (38, 39) befindlicher Teil des Zuschnitts an dem Messtaster (35, 36) anliegt,
e) eine Drehachse der Halterung (33) bzw. der Haltbacken (38, 39) erstreckt sich entlang der Ausrichtkante (41).

2. Vorrichtung nach Anspruch 1, **dadurch gekennzeichnet, dass** der Zuschnitt (10) in Ausgangsstellung in vertikaler Ebene ausgerichtet ist.

3. Vorrichtung nach Anspruch 2, **dadurch gekennzeichnet, dass** der Zuschnitt (10) in Ausgangsstellung mit einer freien Randkante auf einem Stützanschlag (37) abgestützt und ausgerichtet ist.

4. Vorrichtung nach Anspruch 1 oder einem der weiteren Ansprüche, **dadurch gekennzeichnet, dass** die an einem drehbaren Träger angeordneten Haltebacken (38, 39) um eine horizontale Achse drehbar sind.

5. Vorrichtung nach Anspruch 1 oder einem der weiteren Ansprüche, **dadurch gekennzeichnet, dass** die Drehachse der Halterung (33) bzw. der Haltebacken (38, 39) bei Zuschnitten (10) für gerundete oder abgeschrägte Packungskanten (40) auf einen (gedachten) Mittelpunkt einer Rundung (52) oder einer Abschrägung (53) ausgerichtet ist.

6. Vorrichtung nach Anspruch 1 oder einem der weiteren Ansprüche, **dadurch gekennzeichnet, dass** der Messtaster (35, 36) mit drehbaren Tastorganen, insbesondere mit Tastrollen (51) an dem Zuschnitt (10) bzw. an einem zu prüfenden Faltlappen anliegt.

7. Vorrichtung nach Anspruch 1 oder einem der weiteren Ansprüche, **dadurch gekennzeichnet, dass** zur gleichzeitigen Prüfung von Faltlappen bzw. Gruppen von Faltlappen bzw. Teilen des Zuschnitts (10) mehrere Messorgane, nämlich Messtaster (35, 36) nebeneinander angeordnet sind, vorzugsweise in horizontaler Ausrichtung und dass die Messtaster (35, 36) verstellbar sind vorzugsweise durch Verschiebung mittels Getriebe in horizontaler Richtung.

8. Vorrichtung nach Anspruch 1 oder einem der weiteren Ansprüche, **dadurch gekennzeichnet, dass** der Zuschnitt (10) automatisch der Prüfeinheit (31) zuführbar ist, vorzugsweise durch einen Zuschnittförderer mit Saugbändern (67, 68), wobei der Zuschnitt (10) in den Spalt (54) zwischen den Haltebacken (38, 39) in deren Längsrichtung einführbar und auf dem Stützanschlag (37) absetzbar ist.

9. Vorrichtung nach Anspruch 8, **dadurch gekennzeichnet, dass** der Zuschnitt (10) im Bereich der Prüfeinheit (31) durch den Zuschnittförderer bzw. durch dessen Saugbänder (67, 68) in der exakten Prüfstellung gehalten ist.

10. Vorrichtung nach Anspruch 8, **dadurch gekennzeichnet, dass** der zu prüfende Zuschnitt (10) durch ein Entnahmeorgan, insbesondere durch einen Saugkopf (58) mit Saughalter (59) einem Zuschnitt-Magazin (55) entnehmbar und dem Zuschnittförderer bzw. den Saugbändern (67, 68) übertragbar ist.

## Claims

1. Apparatus for measuring the folding behaviour and/or the folding resistance of blanks (10) and their folding flaps delimited by stamped longitudinal or transverse folding lines (24, 19), in particular of blanks (10) made of cardboard for producing cigarette packs of the hinge-lid-box type, in a testing unit (31) with a mount (33) for accommodating the blank (10) between retaining mechanisms, it being possible for a free part of the blank, which is not fixed in the mount (33), to be pivoted relative to the rest of the blank and for the restoring force produced in one or more folding flaps to be measured by means of measuring sensors (35, 36), **characterized by** the following features:
a) the mount (33) comprises two retaining jaws (38, 39), between which a sub-region of the blank (10) is fixed,
b) the mount (33), namely the retaining jaw (39) thereof, has an aligning edge (41), against which the folding line (19, 24) butts during the testing operation,
c) the aligning edge (41) is matched to the shape of the folding line (24) which is to be tested, or to the pack edge (40), namely by way of a recess (42) for a stamped folding line (24) or by way of a rounded portion (52) for measuring blanks (10) with round pack edges (40) or by way of bevelling (53) for measuring blanks (10) with bevelled pack edges (40),
d) the mount (33) can be rotated for carrying out the testing, a part of the blank which is located outside the region of the retaining jaws (38, 39) butting against the measuring sensor (35, 36),
e) an axis of rotation of the mount (33) or of the retaining jaws (38, 39) extends along the aligning edge (41).

2. Apparatus according to Claim 1, **characterized in that** the blank (10) is oriented in a vertical plane in the starting position.

3. Apparatus according to Claim 2, **characterized in that** the blank (10) is supported, and aligned, on a supporting stop (37) by way of a free peripheral edge in the starting position.

4. Apparatus according to Claim 1 or one of the further claims, **characterized in that** the retaining jaws (38, 39), which are arranged on a rotatable support, can be rotated about a horizontal axis.

5. Apparatus according to Claim 1 or one of the further claims, **characterized in that**, in the case of blanks (10) for rounded or bevelled pack edges (40), the axis of rotation of the mount (33) or of the retaining jaws (38, 39) is aligned with an (imaginary) centre point of a rounded portion (52) or of bevelling (53).

6. Apparatus according to Claim 1 or one of the further claims, **characterized in that** the measuring sensor (35, 36) has rotatable sensing mechanisms, in particular sensing rollers (51), butting against the blank (10) and/or against a folding flap which is to be tested.

7. Apparatus according to Claim 1 or one of the further claims, **characterized in that**, for the simultaneous testing of folding flaps or groups of folding flaps or parts of the blank (10), a plurality of measuring mechanisms, namely measuring sensors (35, 36), are arranged one beside the other, preferably in horizontal alignment, and **in that** the measuring sensors (35, 36) can be adjusted, preferably by displacement by means of gear mechanisms in the horizontal direction.

8. Apparatus according to Claim 1 or one of the further claims, **characterized in that** the blank (10) can be fed automatically to the testing unit (31), preferably by a blank conveyor with suction belts (67, 68), it being possible for the blank (10) to be introduced into the gap (54) between the retaining jaws (38, 39), in the longitudinal direction of the latter, and set down on the supporting stop (37).

9. Apparatus according to Claim 8, **characterized in that** the blank (10) is retained in the precise testing position in the region of the testing unit (31) by the blank conveyor or by the suction belts (67, 68) thereof.

10. Apparatus according to Claim 8, **characterized in that** the blank (10) which is to be tested can be removed from a blank magazine (55), and transferred to the blank conveyor or the suction belts (67, 68), by a removal mechanism, in particular by a suction head (58) with a suction holder (59).

## Revendications

1. Dispositif pour mesurer le comportement de pliage respectivement la résistance au pliage de pièces découpées (10) et de leurs pattes pliables délimitées par des lignes de pliage longitudinales ou transversales marquées (24, 19), en particulier de pièces découpées (10) en carton pour la fabrication de paquets de cigarettes du type boîte à rabat, dans une unité de contrôle (31) avec un support (33) destiné à recevoir la pièce découpée (10) entre des organes de maintien, dans lequel une partie libre, non fixée dans le support (33), de la pièce découpée peut pivoter par rapport au reste de la pièce et la force de rappel produite dans une ou plusieurs pattes pliables peut être mesurée au moyen de palpeurs de mesure (35, 36), **caractérisé par** les caractéristiques suivantes:
a) le support (33) se compose de deux mâchoires de maintien (38, 39) entre lesquelles une zone partielle de la pièce découpée (10) est fixée;
b) le support (33), notamment sa mâchoire de maintien (39), présente une arête d'alignement (41), sur laquelle la ligne de pliage (19, 24) est appliquée pendant le déroulement de l'essai;
c) l'arête d'alignement (41) est adaptée à la forme de la ligne de pliage (24) respectivement à l'arête de paquet (40) à contrôler, notamment par un retrait (42) pour une ligne de pliage marquée (24) ou par un arrondi (52) pour la mesure de pièces découpées (10) avec des arêtes de paquet rondes (40) ou par un chanfrein (53) pour la mesure de pièces découpées (10) avec des arêtes de paquet (40) chanfreinées;
d) le support (33) peut tourner pour l'exécution de l'essai, une partie de la pièce découpée se trouvant à l'extérieur de la zone des mâchoires de maintien (38, 39) étant appliquée sur le palpeur de mesure (35, 36);
e) un axe de rotation du support (33) respectivement des mâchoires de maintien (38, 39) s'étend le long de l'arête d'alignement (41).

2. Dispositif selon la revendication 1, **caractérisé en ce que** la pièce découpée (10) en position initiale est alignée dans un plan vertical.

3. Dispositif selon la revendication 2, **caractérisé en ce que** la pièce découpée (10) en position initiale est soutenue et alignée avec une arête de bord libre sur une butée d'appui (37).

4. Dispositif selon la revendication 1 ou une des autres revendications, **caractérisé en ce que** les mâchoires de maintien (38, 39) disposées sur un support rotatif peuvent tourner autour d'un axe horizontal.

5. Dispositif selon la revendication 1 ou une des autres revendications, **caractérisé en ce que** l'axe de rotation du support (33) respectivement des mâchoires de serrage (38, 39) est, pour des pièces découpées (10) avec des arêtes de paquet arrondies ou chanfreinées (40), aligné sur un centre (imaginaire) d'un arrondi (52) ou d'un chanfrein (53).

6. Dispositif selon la revendication 1 ou une des autres revendications, **caractérisé en ce que** le palpeur de mesure (35, 36) s'applique par des organes palpeurs rotatifs, en particulier avec des galets palpeurs (51), sur la pièce découpée (10) respectivement sur une patte pliable à contrôler.

7. Dispositif selon la revendication 1 ou une des autres revendications, **caractérisé en ce que**, pour le contrôle simultané de pattes pliables ou de groupes de pattes pliables ou de parties de la pièce découpée (10), plusieurs organes de mesure, notamment des palpeurs de mesure (35, 36), sont disposés l'un à côté de l'autre, de préférence en alignement horizontal, et **en ce que** les palpeurs de mesure (35, 36) sont réglables de préférence par déplacement au moyen d'une transmission en direction horizontale.

8. Dispositif selon la revendication 1 ou une des autres revendications, **caractérisé en ce que** la pièce découpée (10) peut être fournie automatiquement à l'unité de contrôle (31), de préférence par un transporteur de pièces découpées avec des bandes aspirantes (67, 68), dans lequel la pièce découpée (10) peut être introduite dans la fente (54) entre les mâchoires de maintien (38, 39) dans leur direction longitudinale et appliquée sur la butée d'appui (37).

9. Dispositif selon la revendication 8, **caractérisé en ce que** la pièce découpée (10) est maintenue dans la position de contrôle exacte dans la région de l'unité de contrôle (31) par le transporteur de pièces découpées respectivement par ses bandes aspirantes (67, 68).

10. Dispositif selon la revendication 8, **caractérisé en ce que** la pièce découpée à contrôler (10) peut être prélevée dans un magasin de pièces découpées (55) au moyen d'un organe de prélèvement, en particulier au moyen d'une tête aspirante (58) avec un support aspirant (59), et transférée au transporteur de pièces découpées respectivement aux bandes aspirantes (67, 68).
